# EUROPEAN PATENT APPLICATION

(11) **EP 4 506 447 A1**
(43) Date of publication of application: **12.02.2025**
(21) Application number: 23784262.0
(22) Date of filing: 04.04.2023
(51) Int. Cl.: C12N 5/0775, C12N 5/078

(54) **SERUM REPLACEMENT, PREPARATION METHOD THEREFOR, AND APPLICATION THEREOF**

(30) Priority: 06.04.2022 CN 202210358313
(71) Applicant: Shanghai Wolwo Stem Cell Technology Co., Ltd., Shanghai 200233 (CN)
(72) Inventor: HU, Gengxi, Shanghai 200233 (CN)
(74) Representative: Metten, Karl-Heinz
(86) International application number: PCT/CN2023/086125
(87) International publication number: WO 2023/193699

(57) **Abstract**

The invention provides a serum replacement, a preparation method therefor, and an application thereof. The serum replacement contains plasma and a platelet lysate derived from a bovine fetus or a newborn calf, and can be prepared by means of carrying out platelet lysis on platelet plasma from the bovine fetus or the newborn calf.

## Description

### TECHNICAL FIELD

The present invention relates to a serum replacement, a preparation method therefor, and use thereof.

### BACKGROUND

Stem cells are a kind of pluripotent cells with self-replicating capacity, and can differentiate into various functional cells under certain conditions. A mesenchymal stem cell is one of the members of the stem cell family, exists in various tissues such as hair follicles, umbilical cords, endometria, adipose, and the like, and has the potential for multi-directional differentiation. Such stem cells have the trilineage differentiation potential of mesenchymal stem cells, for example, the potential to be induced to differentiate into osteoblasts, chondroblasts, adipocytes, and the like, and the potential to differentiate into other cell types, and have unique cytokine secretion functions and immunomodulatory and antiinflammatory effects.

Blood is composed of a liquid component, plasma, and blood cells suspended therein, collectively referred to as whole blood. After anticoagulation treatment and centrifugal precipitation of the isolated blood, the obtained liquid that does not contain cell components is plasma. Plasma can be considered as the extracellular matrix of blood, and its main functions are to carry blood cells, transport substances necessary for maintaining life activities and waste products generated in the body, and the like. After the isolated blood is coagulated, the liquid released by the retraction of the blood clot is serum. Serum is plasma that has had fibrinogen and certain clotting factors removed. Its main function is to provide basic nutrients, hormones, various growth factors, and the like. In general, plasma differs from serum mainly in that plasma contains fibrinogen. Platelets are one of the major corporeal components of blood and play an important role in the process of hemostasis. In addition, a large number of factors are released during platelet activation and degranulation, which participate in inflammatory responses and tissue repair.

The efficient culture of stem cells has long been a challenge that researchers in the art aim to overcome, and a culture medium is indispensable during the cell culture. As one of the major components of the stem cell culture medium, animal serum plays an important role in the growth and proliferation of cells. In animal serum, bovine serum is the most widely used, making it one of the important raw materials in pharmaceutical biotechnology products. Bovine serum can be classified into fetal bovine serum, new born calf serum, calf serum, and adult bovine serum according to the time of blood collection. However, animal serum is not only expensive but also of uncontrollable quality, with significant variability in stability between different batches. Therefore, more and more researchers are beginning to develop serum replacements. For example, the patent with publication No. "CN107384856A" discloses a method for preparing a platelet lysate, and the patent with publication No. "CN101486996A" discloses a non-animal-derived cell culture serum replacement. Known serum replacements are all derived from humans and are available in relatively small quantities. Therefore, the development of a serum replacement with diverse sources, high yield, safety, and stability is urgently needed.

### SUMMARY

The present invention adopts a controllable bovine blood source, ensures the traceability of the bovine blood, and implements strict quality control standards. Moreover, by adopting the preparation process for the serum replacement provided by the present invention, a high-quality, safe, and stable serum replacement product can be obtained. The serum replacement product of the present invention meets the quality control standards for new born calf serum in the General Principles of the "Pharmacopoeia of the People's Republic of China" (2020 edition).

In one aspect of the content, the present invention provides a serum replacement, comprising a platelet lysate and plasma, wherein the platelet lysate and the plasma are derived from a fetal bovine or a new born calf. In some embodiments, the serum replacement is composed of a platelet lysate and plasma derived from a fetal bovine or a new born calf. Preferably, the serum replacement is prepared by the method for preparing the serum replacement of the present invention.

In another aspect, the present invention provides a method for preparing a serum replacement, wherein the method comprises platelet lysis of platelet plasma derived from a fetal bovine or a new born calf.

In some embodiments, the method further comprises separating and removing blood cells other than platelets from whole blood of the fetal bovine or whole blood of the new born calf to obtain the platelet plasma; and, optionally, comprises a sterilizing-grade filtration step after the platelet lysis.

In some embodiments, the blood cells other than the platelets are separated and removed by a centrifugation method, preferably differential centrifugation, preferably under low-temperature conditions.

In some embodiments, the platelet lysis is performed using a freeze-thaw method.

The present invention further provides use of the serum replacement of the present invention in culturing a stem cell and in inducing differentiation of a stem cell. The stem cell is, for example, a mesenchymal stem cell, such as a human hair follicle mesenchymal stem cell, a human umbilical mesenchymal stem cell, a human adipose mesenchymal stem cell, a human endometrial mesenchymal stem cell, and the like. Compared with existing animal serum, the serum replacement of the present invention can significantly improve the proliferation capacity and the differentiation potential of stem cells, for example, mesenchymal stem cells.

### BRIEF DESCRIPTION OF THE DRAWINGS

FIG. 1 shows the cell harvest obtained through isolation and culture of primary umbilical mesenchymal stem cells in a culture medium containing fetal bovine serum and a culture medium containing the fetal bovine serum replacement of the present invention.
FIG. 2 shows the cell harvest obtained through isolation and culture of primary hair follicle mesenchymal stem cells in a culture medium containing fetal bovine serum and a culture medium containing the fetal bovine serum replacement of the present invention.
FIG. 3 shows the adipogenic differentiation of hair follicle mesenchymal stem cells induced in a culture medium containing fetal bovine serum and a culture medium containing the fetal bovine serum replacement of the present invention.

### DETAILED DESCRIPTION

Herein, unless otherwise specified, the combination of the various technical features mentioned herein, such as contents, steps, condition parameters, and the like, is not limited to the embodiments and examples specifically described herein, and any other combination of the various technical features also falls within the scope of the present invention.

Herein, unless otherwise specified, a value range expressed by two end values is to be understood as specifically disclosing all real numbers between the two end values and the value ranges formed by combining two of these real numbers. In addition, unless otherwise specified, when a plurality of optional value ranges or values are specified for the same parameter, these end values and values may be arbitrarily combined, and the ranges derived therefrom are intended to be within the scope of the present invention as long as they fall within the largest continuous range specified.

Herein, unless otherwise specified, a value or a value range, whether preceded by "about" or "approximately" or not, encompasses an approximate range equivalent to the value or the value range as understood by those skilled in the relevant art, for example, a range of ±10% around the value or end value, or the range of ±5%, ±3%, ±2%, ±1%, or ±0.5% around the value or end value.

Herein, unless otherwise specified, the meanings of all scientific and technical terms conform to what is commonly known or known in the art, particularly in the field of stem cells, mesenchymal stem cells, and culture media, for example, as those recorded in textbooks, experimental manuals or in the literature of the prior art.

Herein, open-ended expressions such as "comprising", "including" or "containing" mean that elements not explicitly mentioned, for example, other components for the composition and other steps for the method, are not excluded; closed-ended expressions such as "being composed of ......" or "consisting of ......" mean that only the elements explicitly mentioned are included, but that the composition may contain impurities present in usual amounts, and that the method may include steps that are not explicitly mentioned but are substantially inevitable. The open-ended expressions encompass and are to be considered as specifically disclosing situations defined by the closed-ended expressions.

Herein, features, for example, steps, which are arranged with numbers or letters such as "a", "b", ..., "I", "II", ..., "first (1)", "second (2)", unless otherwise specified, have no ordering meaning, but only a distinguishing or counting meaning.

Herein, unless otherwise stated, the conjunction "or" is synonymous with "and/or" and indicates that one or more of the juxtaposed items or situations may be included, unless the elements are incompatible. For example, "derived from a fetal bovine or a new born calf herein means either a fetal bovine source or a new born calf source, including the presence of both sources.

The present invention provides a serum replacement comprising a platelet lysate and plasma derived from a fetal bovine or a new born calf.

Herein, the identification of a fetal bovine or a new born calf is well known to those skilled in the art and is generally defined according to the age of the bovine and whether or not it has been fed. For example, the fetal bovine may be a 5- to 8-month-old bovine embryo in the body of a pregnant female bovine, and the new born calf may be a calf that is within 14 hours of birth and has not been fed. In addition, a calf may be a bovine within 1 year of birth, while an adult bovine may be a bovine of 1 year old or older.

In some embodiments, the serum replacement is composed of a platelet lysate and plasma, but does not exclude impurities present in usual amounts.

Preferably, the serum replacement is prepared by the method for preparing the serum replacement of the present invention described hereinafter, i.e., the serum replacement may be prepared from platelet plasma derived from a fetal bovine or a new born calf through platelet lysis.

To this end, the present invention provides a method for preparing a serum replacement and the serum replacement prepared thereby, wherein the method comprises platelet lysis of platelet plasma derived from a fetal bovine or a new born calf.

In the present invention, "platelet plasma" refers to plasma that contains platelets but does not contain other blood cells such as erythrocytes and leukocytes; the term includes, for example, platelet-rich plasma, platelet concentrate, and the like. For example, the platelet plasma may be prepared by separating and removing blood cells other than platelets (e.g., erythrocytes and leukocytes) from whole blood.

In the present invention, the whole blood used for preparing the platelet plasma may be preferably collected from a fetal bovine or a new born calf. Before the bovine blood is collected, a conventional anticoagulant can be added to the collection container to prevent the bovine blood from coagulating. Commonly used anticoagulants in the art include oxalates, heparin, citrates, ethylenediaminetetraacetic acid (EDTA), and the like. In a preferred embodiment, the anticoagulant is heparin sodium. When collecting the bovine blood, it should be noted that the site of collection needs to be disinfected, and a syringe and a test tube used for collecting the blood must be kept clean and dry.

In order to prepare the platelet plasma, one of the embodiments may take advantage of the different densities of various blood cells and plasma, using a centrifugation technique to separate the different components and obtain the target component. In the present invention, when the platelet plasma is prepared by centrifugal separation, the supernatant after centrifugation is collected to obtain the platelet plasma.

Commonly used centrifugation techniques include sedimentation centrifugation, differential centrifugation, density centrifugation, and the like. Differential centrifugation involves gradually increasing the centrifugal rotation speed or alternately performing high-speed centrifugation and low-speed centrifugation, such that substances with different sedimentation coefficients are precipitated in batches at the bottom of the tube from the mixed solution. In some embodiments, to increase the yield, multiple rounds of centrifugation may be performed; preferably, differential centrifugation with varying rotation speeds may be performed, where, after one or more rounds of low-speed centrifugation, the remaining portion with the supernatant removed is subjected to one or more rounds of high-speed centrifugation, and the collected supernatants are combined. Preferably, the blood cells other than platelets (hereinafter referred to as "other blood cells") can be separated and removed by differential centrifugation.

Preferably, to avoid sample inactivation during preparation, one of the preferred embodiments adopts low-temperature centrifugation (e.g., low-temperature differential centrifugation method) to separate and remove other blood cells. For example, the low temperature may be 10 °C or lower, or 5 °C or lower, such as 4 °C or lower.

The centrifugation speed may be expressed in different units. The rotation speed during low-speed centrifugation is usually expressed by revolutions per minute (rpm), and the rotation speed during high-speed centrifugation is expressed by "g" or "×g". The "g" and "×g" both represent multiples of the gravitational acceleration, which are synonymous and used interchangeably; for example, 1000 g is synonymous with 1000 ×g. The relative centrifugal force represents the ratio of the centrifugal force to the gravitational force, and is equal to the value preceding "g" or "×g"; for example, a centrifugal force of 1000 g or 1000 ×g indicates a relative centrifugal force of 1000.

The present invention may adopt one or more rounds of centrifugation at the same or different centrifugation speeds to separate and remove other blood cells. The centrifugation speed may be 400 g-2100 g, for example, 800 g-2000 g, 400 g-1000 g, or 1800 g-2100 g, and the centrifugation duration may be 10-30 min, for example, 15-20 min, 10-25 min, or 15-30 min. In some embodiments, the centrifugation to separate and remove other blood cells comprises at least one round of centrifugal separation at 400 g-1000 g for a duration of 10-25 min, preferably performed at low temperature, for example, 4 °C.

In some embodiments, other blood cells are removed by differential centrifugation. In some embodiments, the differential centrifugation comprises at least one round of low-speed centrifugation followed by at least one round of high-speed centrifugation. In such embodiments, a relatively low-speed centrifugation condition is set to initially separate the blood cells from the plasma while keeping platelets suspended in the plasma, thereby resulting in a supernatant layer of platelet plasma with retained platelets and a sedimentary layer enriched with other blood cells. Any centrifugation condition that achieves this effect can be considered a "low-speed centrifugation" condition of the present invention. For example, the low-speed centrifugation may be performed at a rotation speed of 400 g-1000 g for a duration of 10-25 min, preferably at a low temperature, such as 4 °C. For example, the centrifugation may be performed at a rotation speed of 400 g, 600 g, 800 g, or 1000 g for a duration of 10 min, 15 min, 20 min, or 25 min. In one of the preferred embodiments, the low-speed centrifugation is performed at a rotation speed of 400 g for a duration of 15 min at a temperature of 4 °C; in another preferred embodiment, the low-speed centrifugation is performed at a rotation speed of 800 g for a duration of 10 min at a temperature of 4 °C. If not used immediately, the supernatants obtained from multiple rounds of low-speed centrifugal separation may be individually or collectively cryopreserved, for example, at - 80 °C to -4 °C.

Meanwhile, in such embodiments, a relatively high-speed centrifugation condition is set to further centrifuge the remaining portion after the supernatant is removed following low-speed centrifugation, allowing for a secondary separation of the incompletely separated plasma from the blood cells. Any centrifugation condition that achieves this effect can be considered a "high-speed centrifugation" condition of the present invention. Thus, completely separated plasma can be prepared. It is generally accepted that the completely separated plasma does not contain platelets.

Herein, the expression "does not contain" a certain substance includes situations where the substance is substantially absent, as understood by those skilled in the art; that is, the substance is present only in minute amounts below the detection limit, below the standard, and/or not affecting the intended use and intended effect of the product in which the substance is present (e.g., the serum replacement of the present invention).

For example, the high-speed centrifugation may be performed at a rotation speed of 1800 g-2100 g for a duration of 15-30 min, preferably at a low temperature, such as 4 °C. For example, the centrifugation may be performed at a rotation speed of 1800 g, 1900 g, 2000 g, or 2100 g for a duration of 15 min, 20 min, 25 min, or 30 min. In one of the preferred embodiments, the high-speed centrifugation is performed at a rotation speed of 2000 g for a duration of 20 min at a temperature of 4 °C; in another preferred embodiment, the high-speed centrifugation is performed at a rotation speed of 2100 g for a duration of 15 min at a temperature of 4 °C. If not used immediately, the supernatants obtained from multiple rounds of high-speed centrifugation may be individually or collectively cryopreserved, for example, at -80 °C to -4 °C.

The platelet lysis may be performed by a repeated thawing and freezing (freeze-thaw) method. The freeze-thaw method allows platelets suspended in plasma to completely rupture and the contents of the platelets to flow out. The number of freeze-thaw cycles is not limited as long as the aforementioned complete rupture effect can be achieved. The number of freeze-thaw cycles may be counted from the first freezing following the initial thawing of a cryopreserved sample, or from the first thawing following the initial freezing of a non-cryopreserved sample. In one of the embodiments of the present invention, after taking the cryopreserved platelet plasma supernatant, the number of freeze-thaw cycles, counted from the first freezing following the initial thawing, is 3-7, and may be, for example, 3, 4, 5, 6, or 7; in one of the preferred embodiments, it is 6, and in another preferred embodiment, it is 4.

When freezing, it is essential to ensure that the supernatant is completely frozen, and that the freezing time and temperature are determined according to actual conditions. Preferably, the freezing time is not less than 8 h. For example, the freezing time may be 8 h, 9 h, 10 h, 12 h, or 15 h. Preferably, the freezing temperature is not higher than -20 °C. For example, the freezing temperature may be -20 °C, -25 °C, -40 °C, -45 °C, -60 °C, -80 °C, -100 °C, or -150 °C. In one of the preferred embodiments, the freezing temperature is -80 °C, and in another preferred embodiment, it is -45 °C.

Preferably, when thawing, it is essential to ensure that the supernatant in the bottle forms an ice-water mixture, for example, a broken ice block with the size of a fingernail, and that the temperature is not increased. In a preferred embodiment, the temperature of the thawed substance is 0 °C.

In the present invention, the purpose of repeated freeze-thaw cycles is to lyse platelets, and the supernatant obtained by low-speed centrifugation is enriched with platelets. Therefore, in a preferred embodiment, the supernatant obtained by low-speed centrifugation is subjected to repeated freeze-thaw cycles, and then mixed with the supernatant obtained by high-speed centrifugation, and the mixture is further subjected to subsequent operations. For the sake of easy operation, the supernatants obtained by low-speed centrifugation and high-speed centrifugation may be mixed and then subjected to the subsequent operations. Therefore, in another preferred embodiment, the supernatants obtained by low-speed centrifugation and high-speed centrifugation are mixed and then subjected to repeated freeze-thaw cycles.

In the present invention, after repeated freeze-thaw cycles, a sterilizing-grade filtration step may also be included. The sterilizing-grade filtration refers to filtration capable of achieving a sterilization effect, and the intended effect and/or actual effect thereof is not necessarily limited to sterilization, and may also include, for example, removal of cell debris, mycoplasma, and other impurities or microorganisms. In one of the preferred embodiments, the filtration is performed using a 0.22 µm filter membrane. In another preferred embodiment, after the filtration using a 0.22 µm filter membrane, a 0.1 µm filter membrane may be selected for re-filtration. The 0.1 µm filter membrane further removes mycoplasma on the basis of sterilization, improving the safety of serum replacement, and has no influence on the performance (e.g., proliferation capacity) of cultured stem cells.

The present invention further provides use of the serum replacement of the present invention in culturing a stem cell. The present invention further provides use of the serum replacement of the present invention in inducing differentiation of a stem cell. The stem cell may include an embryonic stem cell, an adult stem cell, an (induced) pluripotent stem cell, and the like. The pluripotent stem cell is, for example, a mesenchymal stem cell, specifically, such as a human hair follicle mesenchymal stem cell, a human umbilical mesenchymal stem cell, a human adipose mesenchymal stem cell, a human endometrial mesenchymal stem cell, and the like. The adult stem cell may be an adipose stem cell, an endometrial stem cell, a hair follicle stem cell, or an umbilical stem cell. It should be noted that the term "stem cell" in the present invention does not include cells isolated or obtained from human embryos that are more than 14 days post-fertilization or have undergone *in vivo* development. In some embodiments, the induction of differentiation of the stem cell may be, for example, induction of adipogenic differentiation or chondrogenic differentiation of the stem cell. The serum replacement shows a good effect in inducing adipogenic differentiation or chondrogenic differentiation of the stem cell. In some embodiments, the content (volume ratio) of the serum replacement in a culture medium is 5%-15%. Preferably, the content (volume ratio) of the serum replacement in a culture medium is 10%.

In a preferred embodiment, the stem cell is a mesenchymal stem cell, such as a human hair follicle mesenchymal stem cell, a human umbilical mesenchymal stem cell, a human adipose mesenchymal stem cell, a human endometrial mesenchymal stem cell, and the like.

### Examples

The present invention will be further illustrated in detail with reference to the following examples. It is to be understood, however, that these examples are set forth merely for purposes of illustration and are not intended to limit the scope of the present invention.

"SR" is an abbreviation for a serum replacement herein and in the drawings.

### Example 1. Preparation of Serum Replacement

In this example, a fetal bovine serum replacement, a new born calf serum replacement, a calf serum replacement, and an adult bovine serum replacement were prepared using fetal bovine blood, new born calf blood, calf blood, and adult bovine blood, respectively. The fetal bovine blood was collected by cardiac puncture from a fetal bovine in the body of a female bovine that was 8 months pregnant; the new born calf blood was collected from a new born calf born that was within 14 hours of birth and had not been fed; the calf blood was collected from a 6-month-old calf; and the adult bovine blood was collected from 18-month-old adult bovine. The sources of the bovine blood samples are traceable, and strict quality control standards were implemented.

### Collection and transportation of bovine blood

Before the bovine blood was collected, 100 mg of heparin sodium was added to a 500 mL serum bottle to achieve a final concentration of 200 mg/L of heparin sodium; during the collection of bovine blood, the serum bottle was slowly shaken to mix the bovine blood and the sodium heparin uniformly; after the collection was completed, the bovine blood was transported to a serum replacement preparation laboratory at 4 °C.

### Preparation of serum replacement

The collected bovine blood was aliquoted and placed in a biological safety cabinet. The first round of centrifugation at low speed: The centrifugation was performed at 400 g for 15 min at 4 °C. After the centrifugation, the tubes were carefully taken out from the centrifuge. The mixture had been separated into an upper supernatant and a lower pellet, with the pellet consisting of a leukocyte layer adjacent to the upper supernatant and an erythrocyte layer at the bottom. The upper supernatant was collected into a 250 mL serum bottle, labeled as A1, and cryopreserved at -80 °C. The second round of centrifugation at high speed: The pellet, from which the upper supernatant had been removed, was centrifuged at 2000 g for 20 min at 4 °C. The resulting supernatant was then carefully aspirated and placed into a 500 mL serum bottle, labeled as A2, and cryopreserved at -20 °C.

A1 was taken out and equilibrated at room temperature for 10 min, then placed in a 37 °C constant-temperature water bath. It was essential to ensure that the water in the bath covered two-thirds of the bottle's body. A1 was shaken at 80-100 rpm to achieve thawing, with observations made every 10 min. Once an ice-water mixture (0 °C) was formed inside the bottle, the bottle was immediately taken out, concluding the thawing. A1 was then cryopreserved in a -80 °C ultra-low temperature freezer for 8 h or longer until A1 was completely frozen. This process was counted as one freeze-thaw cycle. The freeze-thaw cycle (i.e., freezing and thawing) was repeated 6 times.

After the 6 freeze-thaw cycles were completed, A1 and A2 were thawed separately in a 37 °C constant-temperature water bath, and then mixed uniformly in the biological safety cabinet. Finally, the mixture was filtered through a 0.22 µm filter membrane to prepare a serum replacement (referred to as "SR"). The serum replacement was aliquoted into other serum bottles, and cryopreserved at a temperature of -15 °C or lower for future use.

### Example 2. Culturing Human Hair Follicle Mesenchym al Stem Cells with Serum Replacements from Different Sources

### Obtaining primary human hair follicle mesenchymal stem cells

Intact human hair follicle tissues were obtained and placed separately into 10 cm culture dishes each containing 10 mL of culture medium containing a bovine SR. The culture medium containing the bovine SR was DMEM/F12 culture medium (purchased from Shanghai Basalmedia Technologies Co., Ltd.) supplemented with 1% (v/v) penicillin/streptomycin (purchased from Beyotime Institute of Biotechnology) + 10% fetal bovine SR, new born calf SR, calf SR or adult bovine SR. The fetal bovine SR, the new born calf SR, the calf SR, and the adult bovine SR were prepared by the method described in Example 1. The human hair follicle tissue was carefully placed at the bottom of a 1.5 mL EP tube with the aid of a microscope, the culture medium carried by the human hair follicle tissue was aspirated as far as possible using a pipette gun, and an enzymatic digestion solution was added at 8 µL per hair follicle. The EP tube containing the cell suspension for enzymatic digestion was placed in a 37 °C constant-temperature metal bath for enzymatic digestion for 3 h, with the rotation speed set to 600 rpm (revolutions/minute).

The enzymatic digestion solution was prepared by mixing the following components: 0.05 mL of 20 mg/mL collagenase IV (Sigma-Aldrich), 0.05 mL of 40 mg/mL Dispase II (Sigma-Aldrich), 0.01 mL of 500 mM CaCl₂ (Sigma-Aldrich), and 0.89 mL of HBSS (balanced salt solution, Shanghai Basalmedia Technologies Co., Ltd.).

After the enzymatic digestion was completed, as observed under the microscope, the outer root sheath of the hair follicle was completely digested, and the hair shaft part could not be completely digested. The enzymatic hydrolysate in the EP tube was gently pipetted 20 times using a 20-200 µL pipette gun to ensure uniform mixing. The enzymatic hydrolysate was left to stand for 1 min such that the undigested hair shafts could settle at the bottom of the EP tube. The upper digested cell suspension was then aspirated, which was considered the primary (generation P0) mesenchymal stem cells. An equal amount of AO/PI reagent was added and the mixture was mixed uniformly. The cells were then counted using a Countstar fluorescence cell counter. The use of the automatic cell counter is detailed in the "Standard Operating Procedure for Countstar Rigel S2 Automatic Cell Counter" WWSC-0024-CC.

### Obtaining the first generation of human hair follicle mesenchymal stem cells

In advance, 2 mL of the aforementioned DMEM/F12 culture medium containing 10% fetal bovine SR, new born calf SR, calf SR or adult bovine SR + 1% penicillin/streptomycin was taken out and placed at the bottom of a centrifuge tube. A cell strainer was placed above the wells of a six-well plate. According to the amount of 5 hair follicles/well, 40 µL of the digested cell suspension corresponding to 5 hair follicles was aspirated and filtered through a 100 µm cell strainer. Subsequently, the prepared culture media containing various bovine SRs in the above centrifuge tubes were aspirated and filtered through the cell strainer using the same pipette tips, and this process was repeated 3 times. Then, 200 µL of each of the prepared culture media containing various bovine SRs in the above centrifuge tubes was aspirated using a 20-200 µL pipette gun and used to rinse the EP tube before being filtered through the cell strainer. This rinsing and filtering process was repeated 3 times. Finally, the remaining prepared culture media containing various bovine SRs in the above centrifuge tubes were each filtered through the cell strainer. The six-well plate was placed in a 37 °C cell incubator with 5% CO₂ for culture. After 5 days of static culture, the cells were observed and photographed, and the culture medium was exchanged. The culture medium was exchanged on day 5 and day 7.

The cells were cultured for 10-12 days, and once the cell density in the six-well plate was observed to reach over 80%, the culture medium was discarded. Then, 0.5 mL of TrypLE was added to the six-well plate and left at room temperature for digestion for 3 min. Then, 0.5 mL of the aforementioned DMEM/F12 culture medium containing 10% fetal bovine SR, new born calf SR, calf SR or adult bovine SR + 1% penicillin/streptomycin was added to the corresponding culture bottle using a pipette to terminate digestion. After inoculation, the first generation (generation P1) of human hair follicle mesenchymal stem cells was obtained.

An equal amount of AO/PI reagent was added and the mixture was mixed uniformly. The cells were then counted using a Countstar fluorescence cell counter. The use of the automatic cell counter is detailed in the "Standard Operating Procedure for Countstar Rigel S2 Automatic Cell Counter" WWSC-0024-CC. Amplification factor = number of cells harvested after passaging/number of inoculated cells. In this example, the passaging was strictly performed at a density of 5000 cells/cm², and the area of one six-well plate was 10 cm², i.e., 0.5 × 10⁵ cells were inoculated per six-well plate at a time, so the calculation formula for the amplification factor was "amplification factor = number of cells harvested at a time/(0.5 × 10⁵)".

Following the aforementioned method, hair follicle tissues from two different donor sources were obtained, and the cell harvest and amplification factors for the generation P0 and the generation P1 are shown in Table 1 below.

**Table 1: Assessment of the effect of the fetal bovine SR, the new born calf SR, the calf SR, or the adult bovine SR on hair follicle cell growth**

| Donor | Source of SR | Generation P0 | Generation P1 | |
|---|---|---|---|---|
| | | Cell harvest | Cell harvest | Amplification factor |
| Hair follicle tissue Donor 1 | Fetal bovine SR | 2.45 E+05 | 1.43 E+06 | 28.60 |
| | New born calf SR | 2.18E+05 | 1.41 E+06 | 28.20 |
| | Calf SR | 3.92E+04 | 0.00 E+00 | 0 |
| | Adult bovine SR | 2.32 E+04 | 0.00 E+00 | 0 |
| Hair follicle tissue | Fetal bovine SR | 2.22 E+05 | 6.16 E+05 | 12.32 |
| | New born calf SR | 1.81 E+05 | 5.61 E+05 | 11.22 |
| Donor 2 | Calf SR | 8.27 E+04 | 0.00 E+00 | 0 |
| | Adult bovine SR | 5.62 E+04 | 0.00 E+00 | 0 |

The results above show that the use of the fetal bovine SR and the new born calf SR in the culture media can significantly enhance the proliferation capacity of both primary and passaged mesenchymal stem cells, with the fetal bovine SR being slightly superior to the new born calf SR.

### Example 3. Preparation of Serum Replacement

In this example, the bovine blood was fetal bovine blood. The fetal bovine blood was collected by cardiac puncture from a fetal bovine in the body of a female bovine that was 8 months pregnant.

### Collection and transportation of bovine blood

Before the bovine blood was collected, 100 mg of heparin sodium was added to a 500 mL serum bottle to achieve a final concentration of 200 mg/L of heparin sodium; during the collection of bovine blood, the serum bottle was slowly shaken to mix the bovine blood and the sodium heparin uniformly; after the collection was completed, the bovine blood was transported to a serum replacement preparation laboratory at 4 °C.

### Preparation of serum replacement

The collected bovine blood was aliquoted and placed in a biological safety cabinet. The first round of centrifugation at low speed: The centrifugation was performed at 800 g for 10 min at 4 °C. After the centrifugation, the tubes were carefully taken out from the centrifuge. The mixture had been separated into an upper supernatant and a lower pellet, with the pellet consisting of a leukocyte layer adjacent to the upper supernatant and an erythrocyte layer at the bottom. The upper supernatant was collected into a 250 mL serum bottle, labeled as A1, and stored at 4 °C. The second round of centrifugation at high speed: The pellet, from which the upper supernatant had been removed, was centrifuged at 2100 g for 15 min at 4 °C. The resulting supernatant was then carefully aspirated and placed into a 500 mL serum bottle, labeled as A2. A1 and A2 were mixed uniformly and the mixture was labeled as A3. A3 was cryopreserved at -80 °C.

A3 was taken out and equilibrated at room temperature for 10 min, then placed in a 37 °C constant-temperature water bath. It was essential to ensure that the water in the bath covered two-thirds of the bottle's body. A3 was shaken at 80-100 rpm to achieve thawing, with observations made every 10 min. Once an ice-water mixture (0 °C) was formed inside the bottle, the bottle was immediately taken out, concluding the thawing. A3 was then cryopreserved in a -45 °C ultra-low temperature freezer for 12 h or longer until A3 was completely frozen. This process was counted as one freeze-thaw cycle. The freeze-thaw cycle (i.e., freezing and thawing) was repeated 4 times.

After the 4 freeze-thaw cycles were completed, A3 was placed in a 37 °C constant-temperature water bath for thawing. Finally, thawed A3 was filtered through a 0.22 µm filter membrane to prepare a serum replacement (SR). The serum replacement was aliquoted into other serum bottles, and cryopreserved at a temperature of -15 °C or lower for future use.

### Example 4. Comparison of Serum and Serum Replacement in Culturing Primary Human Mesenchymal Stem Cells

### Comparison of serum and serum replacement in culturing primary human umbilical mesenchymal stem cells

An intact human umbilical cord tissue was obtained, and 1 cm from each end of the umbilical cord was trimmed off using large surgical scissors. The human umbilical cord tissue was then rinsed multiple times to remove blood. The outer amniotic layer was incised along the umbilical vein using small scissors. Tissue forceps were used for blunt dissection from the small opening to completely tear open the amniotic layer, which was then spread flat. The veins and arteries were then removed.

The tissue, from which the blood vessels had been removed, was transferred into a 50 mL centrifuge tube containing 10 mL of digestive enzyme. The tissue was cut into pieces of about 1 mm³ using large surgical scissors. After being sealed with a sealing membrane, the tube was transferred to a 37 °C oscillating shaker at 60-80 rpm and shaken for 2-3 h for digestion.

The digestive enzyme used was prepared according to the following formula: 20 mg/mL collagenase IV (Sigma-Aldrich), 40 mg/mL Dispase II (Sigma-Aldrich), 500 mM CaCl₂ (Sigma-Aldrich), 20 mg/mL hyaluronidase (Beijing Solarbio Science & Technology Co., Ltd.), and 50 mM HEPES (buffer, BBI Life Science).

After the enzymatic digestion was completed, the surfaces of the test tubes were disinfected with 75% ethanol, and then the test tubes were transferred to a biological safety cabinet. Then, 30 mL of balanced salt solution (HBSS) was added for dilution, and the mixture was mixed uniformly and filtered through a 100 µm cell strainer.

The obtained filtrate was aliquoted into 15 mL centrifuge tubes, at about 10 mL per tube, and centrifuged at 400 g for 6 min at room temperature. After the centrifugation was completed, the supernatant was slowly removed. DMEM/F12 culture medium (purchased from Shanghai Basalmedia Technologies Co., Ltd.) containing 10% fetal bovine SR (prepared in Example 3) + 1% penicillin/streptomycin was added at 1 mL per tube to resuspend the pelleted cells. The resuspended cells were then pooled into a single tube and centrifuged at 400 g for 6 min. The supernatant was discarded, and 1 mL of the DMEM/F12 culture medium (purchased from Shanghai Basalmedia Technologies Co., Ltd.) containing 10% fetal bovine SR (prepared in Example 3) + 1% penicillin/streptomycin was used to resuspend the pelleted cells, thus obtaining primary mesenchymal stem cells. An equal amount of AO/PI reagent was added and the mixture was mixed uniformly. The cells were then counted using a Countstar fluorescence cell counter. The use of the automatic cell counter is detailed in the "Standard Operating Procedure for Countstar Rigel S2 Automatic Cell Counter" WWSC-0024-CC. The cell harvest of the primary umbilical mesenchymal stem cells was counted.

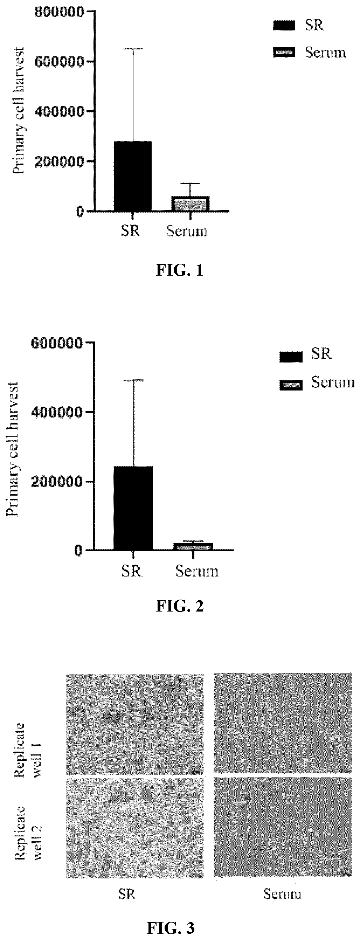

The aforementioned experiment was repeated, with the fetal bovine serum replacement (prepared in Example 3) replaced by fetal bovine serum (purchased from ExCell Bio (Taicang) Co., Ltd.). The primary umbilical mesenchymal stem cells were obtained through isolation and culture, and the cell harvest was counted. The results are shown in FIG. 1.

### Comparison of serum and serum replacement in culturing primary human hair follicle mesenchymal stem cells

Following the method as described in Example 2, DMEM/F12 culture media containing 10% fetal bovine serum (purchased from ExCell Bio (Taicang) Co., Ltd.) and 10% fetal bovine serum replacement (prepared in Example 3), respectively, were used for isolation and culture to obtain primary human hair follicle mesenchymal stem cells, and the cell harvest was counted. The results are shown in FIG. 2.

FIG. 1 and FIG. 2 show that, whether for human umbilical mesenchymal stem cells or human hair follicle mesenchymal stem cells, the use of the fetal bovine serum replacement in the culture medium can enhance the proliferation capacity of primary cells and increase their growth yield.

### Example 5. Comparison of Differentiation Capacity of Stem Cells Cultured with Serum and Serum Replacement

The prepared fetal bovine serum replacement (prepared in Example 3) and commercial fetal bovine serum (purchased from ExCell Bio (Taicang) Co., Ltd.) were used for culture and cell differentiation induction as follows.

### Adipogenic differentiation and staining:

First, hair follicle mesenchymal stem cells were plated. Two wells of the 12-well plate were treated with gelatin and then left to dry at room temperature or in a cell incubator for 15 min. The passaged hair follicle mesenchymal stem cells were harvested. According to the cell concentration, 2.5 × 10⁵ cells were taken and placed into a 15 mL centrifuge tube. Then, 10% fetal bovine serum replacement prepared in Example 3 or 10% commercial fetal bovine serum + human mesenchymal stem cell serum-free culture medium (purchased from Tianjin Haoyang Biological Products Technology Co., Ltd.) were added to achieve a total volume of 2.5 mL, resulting in a final concentration of 1.0 × 10⁵ cells/mL. The mixture was gently inverted 8 times for uniform mixing. The uniformly mixed hair follicle mesenchymal stem cells were immediately plated into the two wells of the pre-coated 12-well plate, with 1 mL of the cell suspension per well. After the plate was shaken 5 times, the cells were observed under a microscope to ensure uniform mixing, and then cultured in a 37 °C incubator.

Then, the adipogenic differentiation induction was performed. When the plated hair follicle mesenchymal stem cells had grown to 90%-100% confluence, the culture medium in the wells was aspirated and discarded in a super clean bench, and 1 mL of adipogenic differentiation induction medium for hair follicle mesenchymal stem cells (MesenCult Adipogenic Differentiation Medium (Human), purchased from STEMCELL Technologies) was added. Every 3 days, the old culture medium was aspirated and discarded, and fresh differentiation induction medium was added, which needed to be pre-warmed in a 37 °C water bath for 15 min before use.

Finally, the cells were stained with oil red O. On the fourteenth day of adipogenic differentiation induction, the culture medium in the wells was aspirated and discarded, and 1 mL of PBS was added. The plate was shaken 3 times in each of four directions (front, back, left, and right), and then the PBS was aspirated and discarded. Then, 4% paraformaldehyde fixative was added at 0.5 mL per well, and the plate was shaken 3 times in each of the four directions to ensure complete wetting of the well bottom, and then left to stand at room temperature for 15 min. After the fixative was aspirated and discarded, 1 mL of PBS was added, the plate was shaken 3 times in each of the four directions, and then the PBS was aspirated and discarded. Subsequently, freshly prepared oil red O staining solution was added at 0.4 mL per well, and the plate was shaken 3 times in each of the four directions to ensure complete wetting of the well bottom, and then left to stand at room temperature for 8 min. After that, 1 mL of isopropanol was added, the plate was shaken to ensure wetting of the well bottom, and then the isopropanol was aspirated and discarded. Subsequently, 1 mL of PBS was added, the plate was shaken 3 times, and then the PBS was aspirated and discarded. Finally, 0.1-0.2 mL of PBS was added, and the staining condition was observed. The results of adipogenic differentiation are shown in FIG. 3, and the results of adipogenic differentiation rate (%) are shown in Table 2.

**Table 2: Adipogenic differentiation rates (%) of stem cells cultured with the serum replacement and the commercial serum**

| | Serum replacement | | Commercial serum | |
|---|---|---|---|---|
| Adipogenic differentiation rate (%) | Replicate well 1 | Replicate well 2 | Replicate well 1 | Replicate well 2 |
| | 35.23 | 18.03 | 0.45 | 0.15 |

As can be seen from Table 2 above, the use of the fetal bovine serum replacement in the culture medium can significantly enhance the adipogenic capacity of the hair follicle mesenchymal stem cells, as compared with the commercial fetal bovine serum.

### Chondrogenic differentiation and detection of expression of chondrogenic differentiation-related genes:

First, the differentiation induction culture was performed. Human hair follicle mesenchymal stem cells were resuspended in 10% fetal bovine serum replacement prepared in Example 3 or 10% commercial fetal bovine serum + human mesenchymal stem cell serum-free culture medium (purchased from Tianjin Haoyang Biological Products Technology Co., Ltd.), and the cell concentration was adjusted to 1 × 10⁷ cells/mL. The cell suspension was added to the inner surface of a 10 cm culture dish lid at the amount of 15 µL/drop in a super clean bench, and 5 mL of PBS was added to the bottom of the culture dish for humidity control. The dish lid with the cell suspension drops was carefully closed and then left to stand in a cell incubator for 24 h, during which the cell suspension drops on the dish lid aggregated into spheres. The microspheres were carefully transferred to a low-adsorption 24-well plate containing 1 mL of chondrogenic differentiation induction medium (MesenCult ACF Chondrogenic Differentiation Basal Medium, purchased from STEMCELL Technologies) using a Pasteur pipette. Every 3 days, the old culture medium was replaced with 1-1.5 mL of fresh chondrogenic differentiation induction medium. On day 3, the cell microspheres were collected into 1.5 mL centrifuge tubes. The cell microspheres were washed twice with 1 mL of PBS, after which the PBS was completely aspirated out. Then, Trizol lysis solution (purchased from Nanjing Vazyme Biotech Co., Ltd.) was added at 50 µL per tube. The cell microspheres were ground for 15 s using a high-speed tissue grinder until they were broken into small fragments. The lysis solution was added at 950 µL per tube, and the mixture was pipetted 5 times using a pipette tip and vortexed for 30 s to ensure complete lysis of the microspheres, resulting in a solution with no visible particles to the naked eye.

Then, the expression of chondrogenic differentiation-related genes was detected. Total RNA was extracted first, then reverse transcribed into cDNA, and finally, gene expression was detected by qPCR.

To the lysate solution of the cell microsphere samples, 100 µL of BCP (1-bromine-3-chloropropane) was added. The mixture was mixed uniformly for 15 s on a vortex mixer, left to stand on ice for 5 min, and then centrifuged at 12000 rpm for 15 min at 4 °C. Each EP tube was then carefully taken out, and about 500 µL of the upper aqueous phase was immediately and gently aspirated into another 1.5 mL RNase-free EP tube, followed by the addition of an equal volume of isopropanol. The mixture was mixed uniformly on the vortex mixer and then frozen at -80 °C for 30 min or longer. After that, the mixture was centrifuged at 12000 rpm for 20 min at 4 °C, and the supernatant was discarded. Then, 1 mL of pre-cooled 75% ethanol was added, and the pellet was washed with inverting the tube. After another centrifugation at 12000 rpm for 5 min at 4 °C, the supernatant was discarded. The pellet was left to dry at room temperature with the lid open. Subsequently, 15-20 µL of DNase/RNase Free H₂O was added, and the mixture was left to stand on ice for 5 min, then mixed uniformly on the vortex mixer, and briefly centrifuged to ensure the pellet was completely dissolved. Then, 1 µL of the total RNA solution was taken to determine the RNA concentration. The detection results were recorded, and 1 µg of RNA was used for subsequent reverse transcription procedures. The reverse transcription was performed at 25 °C for 10 min, 42 °C for 30 min, and 85 °C for 1 min to convert the aforementioned total RNA into cDNA. Primers and the corresponding qPCR system (PerfectStart Green qPCR SuperMix) were prepared to detect the expression of chondrogenic differentiation-related genes ("A comprehensive evaluation strategy for chondrogenic differentiation of human mesenchymal stem cells", Zhang Ke-hua et al., Chinese Journal of New Drugs, 2017, 26(18), 2196-2203). From this, the ratio of up-regulated genes to total chondrogenic differentiation-related genes in human hair follicle mesenchymal stem cells cultured in the culture medium containing the fetal bovine serum replacement or in the culture medium containing the commercial fetal bovine serum was obtained, and the results are shown in table 3 below.

**Table 3: The ratio of up-regulated genes to total chondrogenic differentiation-related genes (%)**

| Days of differentiation induction | Fetal bovine serum replacement | Commercial fetal bovine serum |
|---|---|---|
| 3 days | 29.04 | 26.09 |

Table 3 suggests that the use of the fetal bovine serum replacement in the culture medium can enhance the expression of the chondrogenic differentiation-related genes, indicating an enhancement in chondrogenic differentiation, as compared with the commercial fetal bovine serum.

While specific examples of the present invention have been described, it will be apparent to those skilled in the art that various changes and modifications can be made to the present invention without departing from the spirit and scope of the present invention. Therefore, the appended claims encompass all such changes and modifications that fall within the scope of the present invention.

## Claims

1. A serum replacement, wherein the serum replacement comprises a platelet lysate and plasma, the platelet lysate and the plasma being derived from a fetal bovine or a new born calf; preferably, the serum replacement is composed of the platelet lysate and the plasma; preferably, the fetal bovine is a 5- to 8-month-old bovine embryo in the body of a pregnant female bovine, and the new born calf is a calf that is within 14 hours of birth and has not been fed.

2. The serum replacement according to claim 1, wherein
the serum replacement is prepared from platelet plasma through platelet lysis.

3. The serum replacement according to claim 2, wherein
the platelet plasma is prepared by separation and removal of blood cells other than platelets from whole blood of the fetal bovine or the new born calf; optionally, the serum replacement further has one or more of the following characteristics:
the whole blood of the fetal bovine or the new born calf is anticoagulated;
the separation is performed using a centrifugation method, preferably low-temperature centrifugation; the centrifugation is preferably differential centrifugation; and
the platelet lysis is performed using a freeze-thaw method.

4. A method for preparing the serum replacement according to claim 1, wherein
the method comprises platelet lysis of platelet plasma derived from the fetal bovine or the new born calf.

5. The method according to claim 4, wherein
the method further comprises the following steps:
a) separating and removing blood cells other than platelets from whole blood of the fetal bovine or whole blood of the new born calf to obtain the platelet plasma, wherein preferably, the whole blood of the fetal bovine or the whole blood of the new born calf is anticoagulated; and
b) optionally, after the platelet lysis, performing sterilizing-grade filtration.

6. The method according to claim 5, wherein
in step a), the blood cells other than the platelets are separated and removed by a centrifugation method, preferably low-temperature centrifugation; preferably, the centrifugal separation is performed at a speed of 400 g-2100 g, for example, 400 g-1000 g or 1800 g-2100 g, for a centrifugation duration of 10-30 min, for example, 10-25 min or 15-30 min; preferably, the low temperature is 10 °C or lower, or 5 °C or lower.

7. The method according to claim 6, wherein
the centrifugation method is a differential centrifugation method comprising at least one round of low-speed centrifugation capable of obtaining a supernatant layer of platelet plasma and a sedimentary layer enriched with blood cells other than platelets and at least one round of high-speed centrifugation allowing for a secondary separation of the plasma incompletely separated by the low-speed centrifugation from the blood cells, and comprising combining collected supernatants before the platelet lysis and/or incorporating a supernatant obtained by the high-speed centrifugation after the platelet lysis;
preferably, the differential centrifugation comprises at least one round of low-speed centrifugation at a centrifugation speed of 400 g-1000 g for a duration of 10-25 min, and
at least one round of high-speed centrifugation at a centrifugation speed of 1800 g-2100 g for a duration of 15-30 min.

8. The method according to claim 4, wherein
the platelet lysis is performed using a freeze-thaw method; preferably, the freeze-thaw method has one or more of the following characteristics:
the number of freeze-thaw cycles is 3-7;
a freezing time is not less than 8 h;
a freezing temperature is not higher than -20 °C, and is preferably -80 °C; and
the temperature of a thawed substance is 0 °C.

9. Use of the serum replacement according to any one of claims 1-3 in culturing a stem cell, wherein
the stem cell does not comprise a cell isolated or obtained from a human embryo that is more than 14 days post-fertilization or has undergone *in vivo* development; preferably, the stem cell is a mesenchymal stem cell; more preferably, the stem cell is selected from a human hair follicle mesenchymal stem cell, a human umbilical mesenchymal stem cell, a human adipose mesenchymal stem cell, and a human endometrial mesenchymal stem cell.

10. Use of the serum replacement according to any one of claims 1-3 in inducing differentiation of a stem cell, wherein
the stem cell does not comprise a cell isolated or obtained from a human embryo that is more than 14 days post-fertilization or has undergone *in vivo* development; preferably, the stem cell is a mesenchymal stem cell; more preferably, the stem cell is selected from a human hair follicle mesenchymal stem cell, a human umbilical mesenchymal stem cell, and a human endometrial mesenchymal stem cell; preferably, the differentiation can be, for example, adipogenic differentiation or chondrogenic differentiation.
